# EUROPEAN PATENT SPECIFICATION

(11) **EP 1 596 874 B1**
(45) Date of publication and mention of the grant of the patent: **11.07.2007**
(21) Application number: 04710040.9
(22) Date of filing: 11.02.2004
(51) Int. Cl.: A61K 31/706, A61P 35/00, A61P 35/04

(54) **METHOD FOR TREATING LIVER CANCER BY INTRAHEPATIC ADMINISTRATION OF NEMORUBICIN**
VERFAHREN ZUR BEHANDLUNG VON LEBERKREBS DURCH INTRAHEPATISCHE VERABREICHUNG VON NEMORUBICIN
PROCEDE DE TRAITEMENT DU CANCER DU FOIE PAR ADMINISTRATION INTRAHÉPATIQUE DE LA NÉMORUBICINE

(30) Priority: 26.02.2003 EP 03100470
(43) Date of publication of application: 23.11.2005
(73) Proprietor: Nerviano Medical Sciences S.r.l., 20014 Nerviano MI (IT)
(72) Inventor: VALOTA, Olga, I-20025 Legnano (MI) (IT); PACCIARINI, Maria, Adele, I-20158 Milan (IT)
(74) Representative: Mazzini, Giuseppe
(86) International application number: PCT/EP2004/050112
(87) International publication number: WO 2004/075904

(56) References cited:
- WO-A-00/15203
- WO-A-00/66093
- VASEY PAUL A ET AL: "Phase I clinical and pharmacokinetic study of 3'-deamino-3'-(2-methoxy-4-morpholinyl)dox orubicin (FCE 23762)" CANCER RESEARCH, vol. 55, no. 10, 1995, pages 2090-2096, XP000891501 ISSN: 0008-5472 cited in the application

## Description

### FIELD OF THE INVENTION

The present invention relates to the use of nemorubicin for the treatment of liver cancer. In particular, it refers to a treatment schedule of nemorubicin administered via the intrahepatic artery every 6 weeks to patients with liver cancer.

### BACKGROUND OF THE INVENTION

The present invention relates to the use of nemorubicin for the treatment of a liver cancer; in particular, it refers to the intrahepatic administration of nemorubicin for use in the liver tumor theraphy according to a particular treatment schedule.

Nemorubicin hydrochloride, namely 3'desamino-3'[2(S)methoxy-4-morpholinyl]doxorubicin-hydrochloride (below referred to as nemorubicin only) of formula 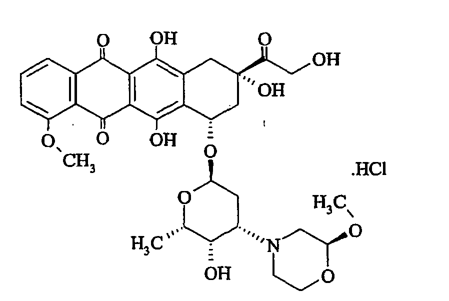 is a doxorubicin derivative obtained with the substitution of the -NH₂ at position 3' in the sugar moiety with a methoxymorpholino group. The compound was synthesized in the course of a research program aimed at identifying new anthracyclines with at least partially novel modes of action, and possessing broad spectrum of activity, including activity on multidrug resistant (mdr) tumors.

Robert J. et al., Cancer Surveys, Vol. 13, 1993, pages 219-252, describe the metabolic pathway of known anthracyclines such as doxorubicin, epirubicin and idarubicin and the rationale for intra-arterial administration of said anthracyclines.

Kirk S. et al., Surgery, Vol. 109, No. 6, 1991, pages 694-697, describe the investigation and treatment of 14 patients with primary hepatocellular carcinoma with infusion of iodized oil and doxorubicin hydrochloride.

Vasey et al., Cancer Research, Vol. 55, No. 10, 1995, pages 2090-2096, describe phase I clinical and pharmacokinetic studies with nemorubicin administered by i.v. bolus injection in patients with refractory solid tumors including patients with liver metastases from colorectal cancer.

Nemorubicin is active *in vitro* and *in vivo* on tumor cells resistant to anthracyclines and presenting the mdr phenotype, this last mechanism being recognized to occur also in man.

No cross-resistance was observed on tumor cells resistant to L-PAM or cDDP, or on cells resistant to Topoisomerase II inhibitors (at-mdr).

Nemorubicin is active after i.p., i.v. or oral administration, with good antitumor activity on murine leukemias, and on solid murine and human tumor models.

The compound differs from most anthracyclines in being highly potent when administered *in vivo*, the optimal i.v. dose being at least 80 fold less than that of doxorubicin. This result, and the observation that the cytotoxic activity of nemorubicin is increased *in vitro* in the presence of mouse, rat and human liver microsomes, suggests that nemorubicin may be transformed into highly cytotoxic metabolite(s).

A well-known pathway of the metabolic transformation of the antitumor anthracyclines in mammals is the side-chain carbonyl group reduction, giving the corresponding 13-dihydro derivative. The reduced derivative of nemorubicin maintains activity *in vitro* and *in vivo* against doxorubicin-resistant models, at doses however 10 fold higher as compared to the parent drug.

The high lipophilicity of the molecule, which confers to the compound the ability to reach high intracellular concentrations and is most likely one of the reasons of its efficacy on resistant models, makes it effective also after oral administration. The oral antitumor efficacy of nemorubicin has been examined in a panel of different tumor types with various schedules of administration. The results demonstrate that the oral treatment with nemorubicin is associated, in all the animal models examined, with an antitumor activity comparable to that observed after intravenous (i.v.) administration. In these models, the effective oral doses of nemorubicin are 1.3-2 fold higher than the effective i.v. doses. In particular, in liver metastases from M5076 murine fibrosarcoma, the best result (doubling of survival time) was achieved with the oral formulation, administered daily for 5 days; the injectable formulation was less effective. This might be a reflection of a different behavior of the drug, due to first pass effect to the liver.

In addition, the liver is a common site of metastasis in many human cancers.

### Primary Liver Cancer

Tumors of the liver are among the most common malignancies in the world. The annual international incidence of the disease is approximately 1 million cases, with a male to female ratio of approximately 4:1. There is a huge geographic variation incidence corresponding to 2/100,000 in North America to 30/100,000 in South East Asia, although these numbers refer often to "total liver cancer", without a differentiation between primary and secondary.

The highest incidence of liver cancer is seen in the Far East and is associated with high endemic hepatitis B carrier rates, contamination of foodstuffs, stored grains, drinking water and soil. Advances in the management of these malignancies will likely depend on immunization strategies for hepatitis B and C and on developing a means of decreasing cirrhosis of any origin. Cirrhosis is frequently associated with HCC, especially in Europe and USA. Systemic chemotherapy is generally disappointing, with response rate averaging less than 20%.

A wide variety of both surgical and nonsurgical therapies have become available for HCC. Surgical resection and orthotopic transplantation are the only curative options, but it is estimated that less than 10% of patients are suitable for this approach and long-term results are poor. The low resectability and the high recurrent rate (40% in five years after surgery), together with the fact that HCC tends to be fatal because of local hepatic progression rather than widespread metastasis, stimulated the development of several locoregional therapeutic approaches, including intra-arterial chemotherapy. Higher response rates appear to be reported for intra hepatic artery (IHA) chemotherapy administered along with embolizing agents, such as LIPIODOL^{®} gel foam and degradable starch microspheres. This approach is increasingly used in the Far East. Anthracyclines (doxorubicin and epirubicin) are widely used in this setting. However, no substantial improvement in survival is obtained with current chemotherapeutic attempts. The need for new effective treatments remains high.

### Secondary Liver Cancer

Liver is a common site of metastasis in many human cancers and hepatic involvement is often the major cause of morbidity and mortality in disseminated malignancy. In particular, the liver, by virtue of the portal venous drainage system, is usually the first - and may be the only - site of metastases in many patients with primary colorectal cancer. Gastric and pancreatic cancers - but also melanoma, lung and breast cancers - may also frequently metastasize to the liver. Metastatic liver tumors are often the first evidence of the progression of a patient's cancer, and particularly in colorectal cancer are the only tumors detected. Colorectal carcinoma is a disease of industrialized nations. It is estimated that in USA over 160,000 new cases were diagnosed yearly and that 75,000 deaths occurred as a result of the advanced disease. Epidemiological studies show that the incidence of colorectal carcinoma is increasing. Involvement of the liver is found in 40-70% of patients with progressive disease and liver is the sole site of initial tumor recurrence in up to 30% of patients with metastatic disease. Left untreated, metastatic lesions to the liver from colorectal cancers are associated with survival of 3 to 24 months.

For patients with isolated liver metastases, surgical resection is the best treatment option, with 20-30% 5-year survival rate. Surgery is only possible in about 10% of cases and it is estimated that up to 25% of patients undergoing surgical resection will recur with metastatic liver cancer.

Palliation with systemic chemotherapy is currently offered to most patients with extensive or multiple liver metastases. To date, systemic 5-fluoruracil (5-FU) plus folinic acid is considered the optimum treatment for metastatic colorectal cancer, yielding response rates of only 20% and overall survival of around 12 months. Irinotecan hydrochloride trihydrate is the standard treatment after failure of 5-FU leucovorin, with a response rate of 15% and median survival time of approximately 9 months.

In case the disease is confined to the liver and it is inoperable, regional intraarterial chemotherapy may be indicated. With the hepatic arterial infusion of 5-FU or of its analogue, 5-fluorodeoxyuridine (FUDR), attempts have been made to maximise the clinical outcome (response rate in up to 50% of cases) but with no substantial effect on survival.

Nemorubicin represents a therapeutic option in the treatment of a liver cancer.

The expectation that nemorubicin is effective in liver neoplasms comes from the findings of phase I and phase IB studies conducted by intravenous route (i.v.), on a total of 197 patients in Europe and United States. During this evaluation, regressions of liver metastases were repeatedly observed in colorectal and renal cancer patients. Tumor shrinkage occurred at doses of 1250 and 1500 mcg/m². The principle toxicities were nausea and vomiting (requiring intravenous antiemetic treatment), myelosuppression and transient elevations in transaminases.

In addition, in a phase II study evaluating i.v. therapy in breast cancer patients with liver metastases previously untreated for the advanced disease, 1 complete response (CR), 3 partial responses (PRs)(2 confirmed and 1 unconfirmed) were observed in liver lesions of 4 out of 6 patients treated at 1500 mcg/m² i.v..

These findings suggest a potential activity of nemorubicin on liver lesions, even in tumor types resistant to conventional chemotherapy such as colorectal cancer and renal cancer.

Strong evidence of antitumor efficacy in liver is also supported by preclinical data. Activity of nemorubicin against liver metastases from M5076 murine reticulosarcoma is higher after oral administration as compared to the i.v. route, suggesting that a first pass effect may favor the efficacy of the compound in liver. In addition, nemorubicin administered orally is more effective on the liver metastases than on the solid primary in the same animal model.

This specific effect on liver metastases might be due to metabolite(s) produced by liver enzymes. This hypothesis is reinforced by several results showing nemorubicin being activated in vitro by liver microsomes to a highly cytotoxic product. This metabolic conversion is believed to occur also in humans.

The hints of activity observed in the current clinical experience, coupled with the activity of nemorubicin in mdr models and in liver metastasis models, raise the expectation of an improved clinical outcome for patients with hepatic neoplastic lesions.

International patent application WO 00/15203 discloses a method for achieving high nemorubicin concentration at the hepatic tumor site, by providing a method for administration of nemorubicin to a patient suffering from a liver tumor, which reduces the nemorubicin amount without decreasing the nemorubicin's antitumor activity at the hepatic tumor site by directly injecting nemorubicin into the hepatic artery.

According to WO 00/15203, nemorubicin is to be administered via the hepatic artery, for example, as an infusion of from about 15 minutes to about 30 minutes every 4 weeks or preferably, as a 5-10 minute bolus every 8 weeks, to adult patients with either a hepatic metastatic cancer, for example, patients with colorectal cancer who have progressed after receiving intravenous chemotheraphy or intrahepatic 5-fluorouracil or 5-fluorodeoxyuridine (FUDR) chemotheraphy, or patients with previously untreated primary liver carcinoma such as, for example, hepatocellular carcinoma or cholangiocarcinoma involving the liver. According to WO 00/15203, nemorubicin is to be administered to a patient in a dosage ranging from, e.g., about 100 mcg/m² to about 1000 mcg/m², preferably from about 100 mcg/m² to about 800 mcg/m², for example, in a dosage of about 200 mcg/m².

It has now been found, and this form the subject of the present invention, that nemorubicin presents a better safety profile and allows the increase of dose intensity over the previous preferred every 8 weeks regimen, when the time interval between treatments is shorten from every 8 weeks to every 6 weeks regimen.

### DESCRIPTION OF THE INVENTION

It is therefore a first object of the present invention the use of nemorubicin for the preparation of a medicament for the treatment of a human liver tumor, which comprises intrahepatic administration of nemorubicin via the hepatic artery in a dosage ranging from 100 mcg/m² to 800 mcg/m², preferably from 200 mcg/m² to 600 mcg/m², for example in a dosage of 200, 400 or 600 mcg/m² every 6 weeks, characterised in that the appropriate dose of nemorubicin, is mixed with a suitable amount of iodized oil, which remains selectively in a liver tumor after its injection through the hepatic artery

Nemorubicin may be administered via the hepatic artery, for example, as a 5-10 minutes infusion every 6 weeks, to adult patients with a liver cancer.

In a still more particular embodiment of the present invention, the appropriate dose of nemorubicin is preferably previously dissolved in saline solution. Preferably, the amount of iodized oil (LIPIODOL^{®}), may vary from 5 ml to 30 ml, depending on the tumor size.

LIPIODOL^{®} is a lipid lymphographic agent, which has been found to remain selectively in liver tumor after its injection through the hepatic artery so it is particularly useful as a carrier of anticancer agents.

The following table illustrates the suitable LIPIODOL^{®} volumes referred to tumor size.

**Table**

| **Tumor size (cm)** | **LIPIODOL^{®} volume (ml)** |
|---|---|
| 2-6 | 5-10 |
| >6-10 | >10-15 |
| >10 | >15-30 |

Tumor size= sum of the longest dimensions of all tumors

For example, for intrahepatic therapy, freeze-dried vials containing 500 mcg of nemorubicin diluted with 1 ml of sterile saline for injection to obtain a nemorubicin concentration of 500 mcg/ml. The appropriate dose of nemorubicin to be given to the patient is optionally mixed with a suitable amount of LIPIODOL^{®}.

The active drug can be administered directly into the lateral entry of an i.v. line inserted into the bung of an intrahepatic potacath lying beneath the upper anterior abdominal wall. The drug can administered, for example, over 5-10 minutes infusion in a suitable volume of normal saline, optionally with LIPIODOL^{®}. Flushing of the device with 10-20 ml of saline can be done after drug infusion to assure that all the drug is given.

Patients who do not have a portacath have a catheter inserted into the hepatic artery by a femoral Seldinger approach and the drug can be infused, for example, over 30 minutes infusion in a volume of 100 ml of normal saline. The catheter is inserted under local anesthesia and can then be removed from the groin, a pressure bandage applied and nursing observations continued overnight in hospital.

According to the present invention, a liver tumor can be a tumor primarily confined to the liver such as, e.g. a hepatocellular carcinoma or a cholangiocarcinoma, or a liver metastasis.

The following Experimental Protocol illustrates the present invention.

### EXPERIMENTAL PROTOCOL

The objective of the study was to shorten the time interval between treatment administrations from every 8 weeks (q8wk) of the protocol as reported in WO 00/15203 to every 6 weeks (q6wk). This was done in the attempt of improving the therapeutic potential of nemorubicin treatment through an increase of the dose intensity of the therapy, simultaneously safeguarding patient safety.

The available results are as follows:
Overall, 13 patients were treated with the q8wk schedule (total 25 cycles administered; doses ranged from 200 to 800 mcg/m²), and 11 patients were treated with the q6wk schedule (total 32 cycles administered; doses were 200 and 600 mcg/m²).
The NCI-Common Toxicity Criteria grading system (CTC Grade), Version 2.0, was used to report toxicities.
**Hematological toxicity:** in both schedules Grade 1-2 thrombocytopenia was observed in most cases, reaching maximum severity Grade 3 in only one patient treated at 600 mcg/m² q8wk after repeated cycles (Table 1). A slight cumulative effect (i.e., increase in frequency but not in severity) was observed after repeated cycles for leucopenia and neutropenia (Tables 2 and 3), mainly with the q8wk schedule.

**Table 1 - Hematological Toxicity - Platelets**

| **Platelets** | | | **Worst CTC Grade by cycle** | | |
|---|---|---|---|---|---|
| **Schedule** | | **Eval. cycles*** | **Grade 1-2 (%)** | **Grade 3 (%)** | **All Grades (%)** |
| 200 | Cycle 1 | 3 | 3 (100%) | - | 3 (100%) |
| mcg/m² | Cycles > 1 | 2 | 2 (100%) | - | 2 (100%) |
| q8wk | All cycles | 5 | 5 (100%) | - | 5 (100%) |
| 400 | Cycle 1 | 3 | 1 (33%) | - | 1 (33%) |
| mcg/m² | Cycles > 1 | 1 | - | - | - |
| q8wk | All cycles | 4 | 1 (25%) | - | 1 (25%) |
| 600 | Cycle 1 | 4 | 4 (100%) | - | 4 (100%) |
| mcg/m² | Cycles > 1 | 4 | 3 (75%) | 1 (25%) | 4 (100%) |
| q8wk | All cycles | 8 | 7 (87%) | 1 (12%) | 8 (100%) |
| 800 | Cycle 1 | 3 | 2 (67%) | - | 2 (67%) |
| mcg/m² | Cycles > 1 | - | - | - | - |
| q8wk | All cycles | 3 | 2 (67%) | - | 2 (67%) |
| 200 | Cycle 1 | 5 | 4 (80%) | - | 4 (80%) |
| mcg/m² | Cycles > 1 | 6 | 6 (100%) | - | 6 (100%) |
| q6wk | All cycles | 11 | 10 (91%) | - | 10 (91%) |
| 600 | Cycle 1 | 6 | 6 (100%) | - | 6 (100%) |
| mcg/m² | Cycles > 1 | 12 | 11 (92%) | - | 11 (92%) |
| q6wk | All cycles | 18 | 17 (94%) | - | 17 (94%) |

| | | | | | |
|---|---|---|---|---|---|
| * Evaluable cycle: a cycle is evaluable if at least 2 assessments exist between days 15-28 (inclusive), or if a Grade 3 or 4 is observed. | | | | | |

**Table 2 - Hematological Toxicity - Leucocytes**

| **Leucocytes** | | | **Worst CTC Grade by cycle** | | |
|---|---|---|---|---|---|
| **Schedule** | | **Evaluable Cycles°** | **Grade 1-2 (%)** | **Grade 3 (%)** | **All Grades (%)** |
| 200 mcg/m² | Cycle 1 | 3 | 2 (67%) | - | 2 (67%) |
| q8wk | Cycles > 1 | 2 | 2 (100%) | - | 2 (100%) |
| | All cycles | 5 | 4 (80%) | - | 4 (80%) |
| 400 mcg/m² | Cycle 1 | 3 | 1 (33%) | - | 1 (33%) |
| q8wk | Cycles > 1 | 1 | - | - | - |
| | All cycles | 4 | 1 (25%) | - | 1 (25%) |
| 600 mcg/m² | Cycle 1 | 4 | 3 (75%) | - | 3 (75%) |
| q8wk | Cycles > 1 | 4 | 4 (100%) | - | 4 (100%) |
| | All cycles | 8 | 7 (87%) | - | 7 (87%) |
| 800 mcg/m² | Cycle 1 | 3 | 2 (67%) | - | 2 (67%) |
| q8wk | Cycles > 1 | 1 | 1 (100%) | - | 1 (100%) |
| | All cycles | 4 | 3 (75%) | - | 3 (75%) |
| 200 mcg/m² | Cycle 1 | 5 | - | 1 * (20%) | 1 (20%) |
| q6wk | Cycles > 1 | 6 | 3 (50%) | - | 3 (50%) |
| | All cycles | 11 | 3 (27%) | 1* (9%) | 4 (36%) |
| 600 mcg/m² | Cycle 1 | 6 | 2 (33%) | - | 2 (33%) |
| q6wk | Cycles > 1 | 12 | 6 (50%) | - | 6 (50%) |
| | All cycles | 18 | 8 (44%) | - | 8 (44%) |

| | | | | | |
|---|---|---|---|---|---|
| * Grade 3 in one patient due to hypersplenism syndrome ° Evaluable cycle: a cycle is evaluable if at least 2 assessments exist between days 15-28 (inclusive), or if a Grade 3 or 4 is observed | | | | | |

**Table 3 - Hematological Toxicity - Neutrophils**

| **Neutrophils** | | | **Worst CTC Grade by cycle** | | |
|---|---|---|---|---|---|
| **Schedule** | | **Evaluable cycles*** | **Grade 1-2 (%)** | **Grade 3-4 (%)** | **All Grades (%)** |
| 200 mcg/m² q8wk | Cycle 1 | 3 | 1 (33%) | - | 1 (33%) |
| | Cycles > 1 | 2 | 2 (100%) | - | 2 (100%) |
| | All cycles | 5 | 3 (60%) | - | 3 (60%) |
| 400 mcg/m² q8wk | Cycle 1 | 3 | 1 (33%) | - | 1 (33%) |
| | Cycles > 1 | 1 | - | - | - |
| | All cycles | 4 | 1 (25%) | - | 1 (25%) |
| 600 mcg/m² q8wk | Cycle 1 | 4 | - | - | - |
| | Cycles > 1 | 4 | 2 (50%) | - | 2 (50%) |
| | All cycles | 8 | 2 (25%) | - | 2 (25%) |
| 800 mcg/m² q8wk | Cycle 1 | 3 | 1 (33%) | - | 1 (33%) |
| | Cycles > 1 | 1 | - | - | - |
| | All cycles | 4 | 1 (25%) | - | 1 (25%) |
| 200 mcg/m² q6wk | Cycle 1 | 5 | 2 (40%) | - | 2 (40%) |
| | Cycles > 1 | 6 | 2 (33%) | - | 2 (33%) |
| | All cycles | 11 | 4 (36%) | - | 4 (36%) |
| 600 mcg/m² q6wk | Cycle 1 | 6 | 1 (17%) | - | 1 (17%) |
| | Cycles > 1 | 12 | 2 (17%) | - | 2 (17%) |
| | All cycles | 18 | 3 (17%) | - | 3 (17%) |

| | | | | | |
|---|---|---|---|---|---|
| * Evaluable cycle: a cycle is evaluable if at least 2 assessments exist between days 15-28 (inclusive), or if a Grade 3 or 4 is observed | | | | | |

**Liver toxicity (Table 4):** although Grade 1-2 transaminases elevation (due to all causes, including tumor-related events) seemed to increase in frequency in the q6wk schedule, when considering the most clinically relevant events (i.e., grade 3-4 events), frequency of transaminitis was lower in the q6wk than in the q8wk schedule, allowing administration of repeated cycles.

**Table 4 - Liver Toxicity**

| **Schedule** | **Evaluable cycles°** | **Worst CTC Grade by cycle (increased of at least one Grade versus baseline Grade)** | | |
|---|---|---|---|---|
| | | **Grade 1-2 (%)** | **Grade 3-4 (%)** | **All Grades (%)** |
| **SGOT** | | | | |
| 200 mcg/m² q8wk | 5 | 3 (60%) | - | 3 (60%) |
| 400 mcg/m² q8wk | 4 | 1 (25%) | 3 (75%) | 4 (100%) |
| 600 mcg/m² q8wk | 7 | 2 (28%) | 2* (28%) | 4 (57%) |
| 800 mcg/m² q8wk | 5 | 1 (20%) | 3 (60%) | 4 (80%) |
| 200 mcg/m² q6wk | 12 | 8 (67%) | 1 (8%) | 9 (75%) |
| 600 mcg/m² q6wk | 17 | 14 (82%) | 2 (12%) | 16 (94%) |

| **SGPT** | | | | |
|---|---|---|---|---|
| 200 mcg/m² q8wk | 5 | 4 (80%) | - | 4 (80%) |
| 400 mcg/m² q8wk | 4 | 2 (50%) | 1 (25%) | 3 (75%) |
| 600 mcg/m² q8wk | 7 | 1 (14%) | 3* (43%) | 4 (57%) |
| 800 mcg/m² q8wk | 6 | - | 5 (83%) | 5 (83%) |
| 200 mcg/m² q6wk | 12 | 7 (58%) | - | 7 (58%) |
| 600mcg/m²q6wk | 18 | 14 (78%) | 2 (11%) | 16 (89%) |

| **Total Bilirubin** | | | | |
|---|---|---|---|---|
| 200 mcg/m² q8wk | 5 | 5 (100%) | - | 5 (100%) |
| 400 mcg/m² q8wk | 4 | 2 (50%) | - | 2 (50%) |
| 600 mcg/m² q8wk | 7 | 7 (100%) | - | 7 (100%) |
| 800 mcg/m² q8wk | 5 | 3 (60%) | 1 (20%) | 4 (80%) |
| 200 mcg/m² q6wk | 12 | 10 (83%) | - | 10 (83%) |
| 600 mcg/m² q6wk | 18 | 13 (72%) | 1 (5%) | 14 (77%) |

| | | | | |
|---|---|---|---|---|
| * Excluding one case of grade 4 SGOT/SGPT attributed to iodinated oil overdose ° Evaluable cycle: a cycle is evaluable if at least 2 assessments exist between days 2-16 (inclusive), or if Grade 3 or 4 is observed. | | | | |

Efficacy: Overall, partial tumor responses (PRs) were observed in 6/24 treated patients (25%). The tumor response was confirmed at least 4 weeks apart in 3 cases (at 200 mcg/m² q8wk and at 200 and 600 mcg/m² q6wk; one case each) and was unconfirmed in the other 3 cases (at 200, 600 and 800 mcg/m² q8wk; one case each). The six responses are commented here below.

### SCHEDULE q8wk

### (number of treated patients =13)

### 200 mcg/m² dose level

Unconfirmed PR was observed in one patient (American Joint Committee on Cancer, AJCC, stage II), who had one measurable lesion in the right liver lobe at study entry. After 2 cycles PR was achieved (85.9% decrease in tumor area versus baseline); the response was not subsequently confirmed due to the appearance of a lesion in the non-perfused left lobe, although it was confirmed in the perfused right lobe. Based on this, the patient was withdrawn from therapy. At the last follow-up (more than 11 months later), the lesion in the right lobe was still in PR and the lesion appeared in the left lobe had not increased in size. Overall, the PR in the original lesion (right lobe) lasted, overall, 12+ months.

Confirmed PR was reported in one patient (AJCC stage III), who presented at study entry 2 lesions in the right liver lobe (one measurable and one evaluable). During the treatment period, only the measurable lesion was monitored, showing a shrinkage of 78.45% versus baseline after 1 cycle. In the second cycle, the lesion area further decreased (assessment performed 26 days after the assessment of cycle 1; 95.3% reduction versus baseline) and the tumor response was regarded as downstaging, i.e. the tumor was made resectable. However, the patient refused surgery and went off study. At the last contact, 8 months later, the patient was alive, no other antitumor therapy had been started and no additional tumor assessment had been performed.

### 600 mcg/m² dose level

One patient (AJCC stage III), enrolled in the study with one measurable lesion in the right liver lobe, was judged to be in PR after one cycle (54.3% decrease of lesion area versus baseline). The tumor response was unconfirmed in cycle 2 due to the appearance of a small lesion in the left non perfused lobe, although it was confirmed in the perfused right lobe (68.2% decrease in tumor area versus baseline). Based on this, the treating physician judged appropriate to administer an additional cycle (cycle 3), at the end of which disease progression was observed in both lesions; the patient was withdrawn from the study and started another antitumor therapy. The PR in the original lesion (right lobe) lasted, overall, about 3 months.

### 800 mcg/m² dose level

Unconfirmed PR was observed in one patient (AJCC stage III), who had one measurable lesion in the right liver lobe at study entry. PR was achieved after 3 cycles, with a 52% decrease of the tumor area versus baseline. At the end of the same cycle the patient was taken off study and started a conventional intrahepatic artery therapy.

### SCHEDULE q6wk

### (number of treated patients =11)

### 200 mcg/m² dose level

Confirmed PR was reported in one patient (AJCC stage II), who presented at study entry one measurable lesion in the right liver lobe. PR was achieved after 2 cycles, with 64% tumor shrinkage versus baseline; at that time the patient withdrawn his consent and went off study. However, he agreed to go back to the hospital after 5 weeks for a CT scan assessment, based on which the PR was confirmed (70% lesion area reduction versus baseline). At the last contact, 2 months later, the patient was alive and had not started any other antitumor therapy.

### 600 mcg/m² dose level

Confirmed and long-lasting PR was observed in one patient (AJCC stage III), who had 3 lesions in the right liver lobe (two small evaluable lesions and one measurable lesion) at study entry. The overall tumor response was PR since the first treatment cycle, with a 48.4% reduction of the measurable lesion area and disappearance of the two small lesions. The tumor shrinkage was confirmed in the subsequent 2 cycles (78.2% reduction of measurable lesion area in cycle 3 versus baseline), at the end of which the treating physician planned to take the patient off study and proceed with alcohol perfusion. MRI performed in the third cycle showed that the original tumor was still supported by a small vessel, then justifying two additional nemorubicin administrations. After a total of 5 cycles, the patient was withdrawn from the trial still in PR, further confirmed 2 months later at the last follow up visit. Overall, the PR lasted 8+ months.

**Conclusion**: In the q6wk schedule efficacy is very well maintained, with two confirmed PR out of 11 treated patients, versus one confirmed PR out of 13 treated patients in the q8wk schedule.

As for safety, the most relevant effect of the compound is the induction of mild-moderate thrombocytopenia and leucopenia. The available data indicate that the q6wk schedule does not increase the frequency of thrombocytopenia and leucopenia after repeated cycles with respect to the q8wk schedule and can be therefore considered safely administrable. This is particularly relevant due to the fact that, in patients with hepatocellular carcinoma, platelets and white blood cells count decrease as a consequence of disease-associated portal hypertension and hypersplenism.

Regarding the effect on liver transaminases, the q6wk schedule is definitely not worse than the q8wk schedule in producing severe transaminitis.

Therefore, the q6wk schedule represents and advantage over the q8wk since ensures good tolerability and allows the increase of dose intensity, being potentially more beneficial for the patients, as suggested by the two confirmed partial remissions (lasting up 8 months in one case).

## Claims

1. Use of nemorubicin for the preparation of a medicament for the treatment of a human liver tumor, which comprises intrahepatic administration of nemorubicin via the hepatic artery every 6 weeks in a dosage ranging from 100 mcg/m² to 800 mcg/m², **characterized in that** the appropriate dose of nemorubicin is mixed with a suitable amount of iodized oil, which remains selectively in a liver tumor after its injection through the hepatic artery.

2. Use according to claim 1, wherein nemorubicin is to be administered in a dosage ranging from 200 mcg/m² to 600 mcg/m².

3. Use according to claim 1, wherein nemorubicin is to be administered in a dosage of 200 mcg/m².

4. Use according to claim 1, wherein nemorubicin is to be administered in a dosage of 400 mcg/m².

5. Use according to claim 1, wherein nemorubicin is to be administered in a dosage of 600 mcg/m².

6. Use according to claim 1, wherein nemorubicin is to be administered as a 5-10 minutes infusion.

7. Use according to claim 1, wherein nemorubicin is to be administered as a 30 minutes infusion.

8. Use according to claim 1, wherein the appropriate dose of nemorubicin is to be previously dissolved in saline solution.

9. Use according to claim 1, wherein the amount of iodized oil may vary from 5 ml to 30 ml, depending on the tumor size.

10. Use according to claim 1 wherein the liver tumor is primarly confined to the liver.

11. Use according to claim 1, wherein the liver tumor is a liver metastasis.

## Patentansprüche

1. Verwendung von Nemorubicin zur Herstellung eines Medikaments zur Behandlung eines humanen Lebertumors, die die intrahepatische Verabreichung von Nemorubicin durch die Leberarterie jede 6. Woche in einer Dosierung im Bereich zwischen 100 mcg/m² und 800 mcg/m² umfasst, **dadurch gekennzeichnet, dass** die geeignete Dosis von Nemorubicin mit einer passenden Menge an jodiertem Öl gemischt wird, das selektiv in einem Lebertumor nach dessen Injektion durch die Leberarterie verbleibt.

2. Verwendung nach Anspruch 1, wobei Nemorubicin in einer Dosierung im Bereich zwischen 200 mcg/m² und 600 mcg/m² zu verabreichen ist.

3. Verwendung nach Anspruch 1, wobei Nemorubicin in einer Dosierung von 200 mcg/m² zu verabreichen ist.

4. Verwendung nach Anspruch 1, wobei Nemorubicin in einer Dosierung von 400 mcg/m² zu verabreichen ist.

5. Verwendung nach Anspruch 1, wobei Nemorubicin in einer Dosierung von 600 mcg/m² zu verabreichen ist.

6. Verwendung nach Anspruch 1, wobei Nemorubicin als 5-bis 10-Minuten-Infusion zu verabreichen ist.

7. Verwendung nach Anspruch 1, wobei Nemorubicin als 30-Minuten-Infusion zu verabreichen ist.

8. Verwendung nach Anspruch 1, wobei die geeignete Dosis von Nemorubicin zuvor in Kochsalzlösung zu lösen ist.

9. Verwendung nach Anspruch 1, wobei die Menge an jodiertem Öl, abhängig von der Tumorgröße, zwischen 5 ml und 30 ml variieren kann.

10. Verwendung nach Anspruch 1, wobei der Lebertumor primär auf die Leber beschränkt ist.

11. Verwendung nach Anspruch 1, wobei der Lebertumor eine Lebermetastase ist.

## Revendications

1. Utilisation de némorubicine pour la préparation d'un médicament pour le traitement d'une tumeur hépatique humaine, qui comprend l'administration intrahépatique de némorubicine par l'artère hépatique toutes les 6 semaines en un dosage allant de 100 mcg/m² à 800 mcg/m², **caractérisée en ce que** la dose appropriée de némorubicine est mélangée avec une quantité appropriée d'huile iodée, qui reste sélectivement dans une tumeur hépatique après son injection par l'artère hépatique.

2. Utilisation selon la revendication 1, dans laquelle la némorubicine doit être administrée en un dosage allant de 200 mcg/m² à 600 mcg/m².

3. Utilisation selon la revendication 1, dans laquelle la némorubicine doit être administrée en un dosage de 200 mcg/m².

4. Utilisation selon la revendication 1, dans laquelle la némorubicine doit être administrée en un dosage de 400 mcg/m².

5. Utilisation selon la revendication 1, dans laquelle la némorubicine doit être administrée en un dosage de 600 mcg/m².

6. Utilisation selon la revendication 1, dans laquelle la némorubicine doit être administrée sous la forme d'une perfusion de 5 à 10 minutes.

7. Utilisation selon la revendication 1, dans laquelle la némorubicine doit être administrée sous la forme d'une perfusion de 30 minutes.

8. Utilisation selon la revendication 1, dans laquelle la dose appropriée de némorubicine doit être préalablement dissoute dans une solution saline.

9. Utilisation selon la revendication 1, dans laquelle la quantité d'huile iodée peut varier de 5 ml à 30 ml, en fonction de la taille de la tumeur.

10. Utilisation selon la revendication 1, dans laquelle la tumeur hépatique est principalement confinée au foie.

11. Utilisation selon la revendication 1, dans laquelle la tumeur hépatique est une métastase hépatique.
